# EUROPEAN PATENT APPLICATION

(11) **EP 1 743 896 A2**
(43) Date of publication of application: **17.01.2007**
(21) Application number: 06012704.0
(22) Date of filing: 21.06.2006
(51) Int. Cl.: C07D 471/04

(54) **A process for the purification of imiquimod**

(30) Priority: 08.07.2005 IT MI20051292
(71) Applicant: Dipharma S.p.A., 33036 Mereto di Tomba (Udine) (IT)
(72) Inventor: Allegrini, Pietro, 20097 San Donato Milanese (MI) (IT); Razzetti, Gabriele, 20099 Sesto S. Giovanni(MI) (IT); Bologna, Alberto, 27018 Vidigulfo (PV) (IT); Magrone, Domenico, 20128 Milano (IT); Ventimiglia, Gianpiero, 20092 Cinisello Balsamo (MI) (IT)
(74) Representative: Minoja, Fabrizio

(57) **Abstract**

Crystalline forms of imiquimod, a process for the preparation thereof and the use thereof in the purification of imiquimod.

## Description

The present invention relates to a process for the purification of imiquimod, salts and crystalline forms thereof.

### TECHNOLOGICAL BACKGROUND

Imiquimod, 1-isobutyl-1H-imidazo[4,5-c]quinolin-4-ylamine, known from U.S. 4,689,338, of formula 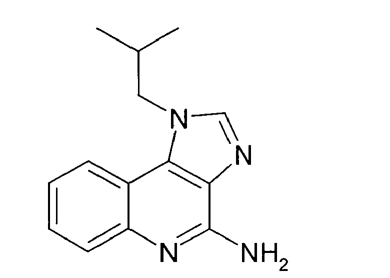 is used in therapy as an antiviral and/or immunomodulating agent.

The processes used for its preparation usually do not provide highly pure imiquimod so that, for regulatory requirements to be fulfilled, imiquimod has to subjected to a subsequent purification process. For example, US 2004/0063743 discloses a process for the purification of imiquimod comprising the transformation of the free base into the hydrochloride, the subsequent treatment with sodium bisulfite and charcoal in water and the final treatment with 26% NH₃ to obtain imiquimod free base.

There is therefore the need for an alternative process for the purification of imiquimod, which makes use of inexpensive, easy-to-use reagents.

### SUMMARY OF THE INVENTION

It has now been found a process which comprises the recovery of imiquimod as an organic acid addition salt and provides a simple way to obtain imiquimod free base with a suitable purity to fulfill regulatory requirements.

### BRIEF DESCRIPTION OF THE FIGURE

The novel crystalline forms were characterized with the known XRPD technique (X-ray powder diffraction). X-ray diffraction spectra (XRPD) were recorded with an APD 2000 θ/θ automatic diffractometer for powders and liquids (Ital-Structures), under the following conditions: radiation CuKα (X = 1.5418 A), scanning with angular interval 2-40°, with angular step of 0.03° for 1 sec.
Figure 1. Spectrum XRPD of imiquimod salt formate.
Figure 2. Spectrum XRPD of imiquimod free base.

According to the invention, the statement that a sample of particles has mean diameter, referred to as D[4,3], higher than X µm, means that the mean of the volumes of the particles forming the sample is higher than the volume of a spherical particle with X diameter.

The term "particle" means a single entity, both as a single and geminate crystal.

Particle size, namely "D[4,3]" mean diameter, was determined with the known laser light scattering technique using a Malvern Mastersizer MS1 instrumentation under the following operative conditions:
- 300RF mm lens with of 2.4 mm laser beam length;
- sample of 500 mg dispersed in 10 ml of hexane (ACS reagent) with 1% SPAN 85^{®}, without presonication, and 2500 rpm stirring rate.

The samples water content was determined by the known Karl - Fischer technique.

### DETAILED DISCLOSURE OF THE INVENTION

Object of the present invention is a process for the purification of imiquimod comprising:
- the preparation of a dispersion of imiquimod free base in an organic polar protic solvent;
- the reaction of imiquimod free base with a mono- or poly-carboxylic organic acid;
- the separation and recovery of the resulting addition salt;
- the reaction of the addition salt with a basic agent to obtain imiquimod free base; and
- the separation and recovery of imiquimod free base.

An organic polar protic solvent is for example a C₁-C₄ alkanol, typically selected from methanol, ethanol, 1-propanol, 2-propanol, 1-butanol and 2-butanol, preferably from methanol and ethanol, more preferably methanol.

A mono- or poly-carboxylic organic acid can be for example a mono-, bi-, tri- or tetra-carboxylic acid, for example, formic, acetic, propionic, oxalic, citric, tartaric, malic, fumaric, malonic or maleic acid. The acid is preferably a monocarboxylic acid, in particular formic acid or acetic acid, more preferably formic acid.

The concentration of imiquimod free base in the starting dispersion can typically range from 5 to 15%, while the organic acid is added in a molar ratio to the base approx. ranging between 1:1 and 10:1, preferably between 4:1 and 6:1.

The temperature of the dispersion of imiquimod free base in the organic solvent preferably ranges from 60°C to the boiling temperature of the solvent used. After the reaction with the organic acid, the dispersion is cooled to separate the corresponding addition salt, which can be recovered according to a conventional techniques, such as filtration or centrifugation, followed by washing, preferably with the same solvent as used in the preparation and drying steps.

The resulting imiquimod addition salts with mono- or poly-carboxylic organic acids are novel compounds and are an object of the present invention. Examples of said salts are those with a mono-, bi-, tri- or tetra-carboxylic acid, for instance formic, acetic, propionic, oxalic, citric, tartaric, malic, fumaric, malonic or maleic acid; preferably with a monocarboxylic acid, in particular formic acid or acetic acid, more preferably formic acid; in particular in a crystalline form.

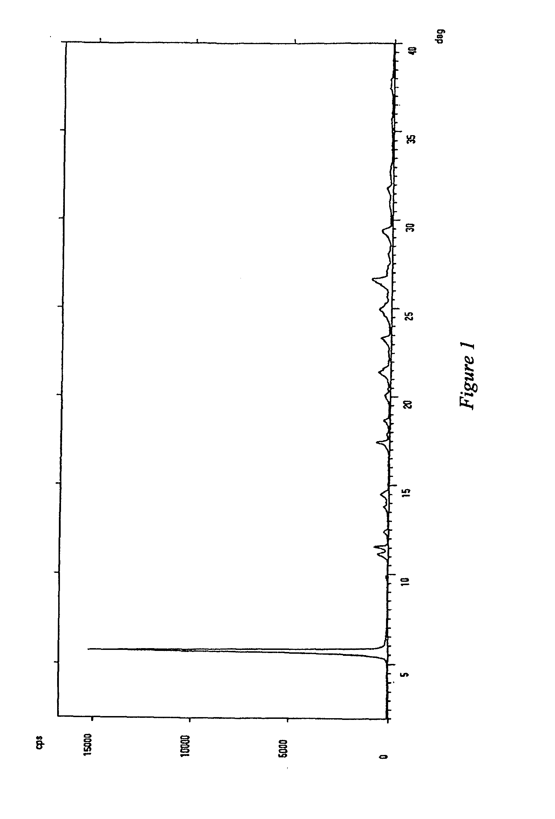

Particularly preferred is imiquimod formate in the crystalline form having a XRPD spectrum substantially as reported in Figure 1, wherein the most intense diffraction peaks fall at 5.72; 11.15; 11.54; 17.42; 21.38; 24.95 in 2θ.

The resulting addition salt is contacted with a basic agent in a polar solvent, to obtain imiquimod free base. According to a preferred aspect, a dispersion of an imiquimod addition salt in a polar solvent is slowly added with the basic agent in equimolar amounts and at a temperature around or equal to the reflux temperature.

The basic agent may be any base commonly used for making a free base. Examples of basic agents are inorganic bases, such as ammonia, either gaseous or in aqueous solution, and sodium hydroxide, either solid or dissolved in water, in particular sodium hydroxide in approx. 50% aqueous solution.

A polar solvent is typically water or an organic polar protic solvent as defined above, preferably water.

Imiquimod free base can be recovered by known techniques, such as filtration or centrifugation, followed by washing, preferably with the same solvent as used in the preparation and drying steps. According to the process of the invention, imiquimod free base is obtained in a purity equivalent to or higher than 99.5%, typically equivalent to or higher than 99.9%, and with a potentiometric titre ranging from 99 to 101%.

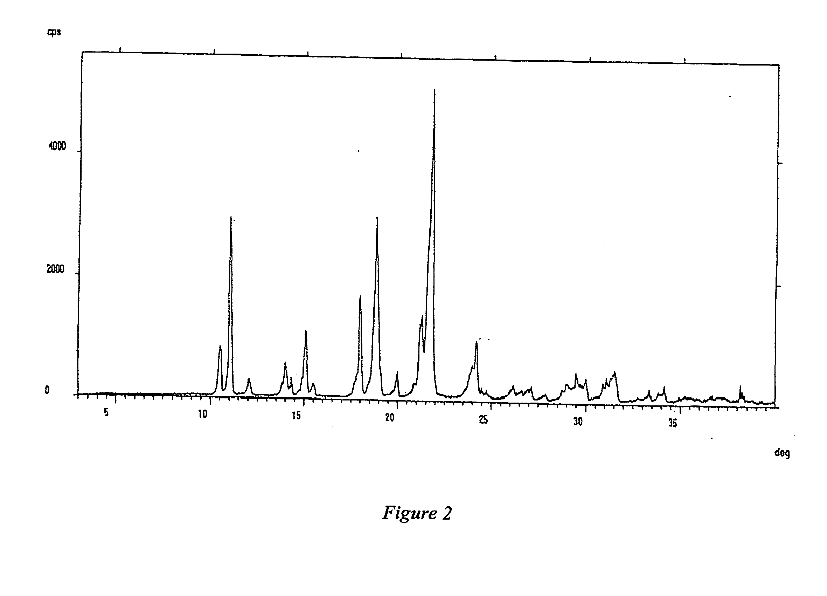

The resulting imiquimod free base is in the form of particles having an XRPD spectrum substantially illustrated in Figure 2, wherein the most intense diffraction peaks fall at 11.04; 17.97; 18.81; 21.24; 21.78; 24.18 in 2θ. Said particles typically have a D[4,3] mean diameter lower than 50 µm, preferably around 30 µm or lower.

If desired, after completion of the process described above, the particles can be subjected to a conventional fine grinding or micronisation step, to obtain particles having lower mean D[4,3] diameter, typically of 5 µm or lower, preferably of 2 µm or lower.

Imiquimod having said particle size distribution is particularly suitable for the formulation in pharmaceutical forms for the topical administration.

The following examples illustrate the invention.

### EXAMPLE 1. Preparation of imiquimod formate salt

A 1 L 4-necked round-bottom flask, equipped with mechanical stirrer, reflux condenser and thermometer, is loaded with 127 g of imiquimod free base, 127 g of 99% formic acid and 1270 ml of methanol. The dispersion is refluxed and subsequently hot filtered through Celite (25 g) to remove insolubles. After washing the celite cake with 150 ml of hot methanol, mother liquors and washings are combined and concentrated to a weight of 270 g. The resulting concentrate is cooled at a temperature of 0°C, after 20 minutes the resulting solid is filtered with suction, washed with methanol at 5°C (2 x 70 ml) and dried in a static dryer under vacuum at a temperature of 60°C to constant weight, thereby obtaining 102 g of imiquimod formate as yellow crystals, having an XRPD spectrum substantially as illustrated in Figure 1, wherein the most intense diffraction peaks fall at 5.72; 11.15; 11.54; 17.42; 21.38; 24.95 in 2θ.

By proceeding analogously the imiquimod salt with acetic, propionic, oxalic, citric, tartaric, malic, fumaric, malonic and maleic acid is obtained.

### EXAMPLE 2. Preparation of imiquimod free base

A 500 ml 4-necked round-bottom flask, equipped with mechanical stirrer, reflux condenser and thermometer, is loaded with 41 g of imiquimod salt formate and 300 ml of water. The dispersion is refluxed, added with 4 g of active charcoal, then hot filtered through Celite. The cake is thoroughly washed with 50 ml of hot water. Mother liquors and washings are combined and alkalinized to pH 11 with 50% NaOH, then cooled to room temperature to precipitate a white solid. This is filtered with suction, washed with water (1 x 30 ml) and methanol (1 x 30 ml), then dried to constant weight in a static dryer under vacuum at a temperature of 60°C, thereby obtaining 19 g of imiquimod free base as a white crystalline product, having HPLC purity higher than 99.5%, potentiometric titre ranging from 99 to 101%, water content according to Karl-Fischer equivalent to or lower than 0.05%, an XRPD spectrum substantially as illustrated in Figure 2, wherein the most intense diffraction peaks fall at 11.04; 17.97; 18.81; 21.24; 21.78; 24.18 in 2θ, and D[4,3] diameter mean of approx. 20 µm.

## Claims

1. A process for the purification of imiquimod comprising:
- the preparation of a dispersion of imiquimod free base in an organic polar protic solvent;
- the reaction of imiquimod free base with a mono- or poly-carboxylic organic acid;
- the separation and recovery of the resulting addition salt;
- the reaction of the addition salt with a basic agent to obtain imiquimod free base; and
- the separation and recovery of imiquimod free base.

2. A process as claimed in claim 1, wherein the protic polar organic solvent is a C₁-C₄ alkanol.

3. A process as claimed in claim 1, wherein the carboxylic acid is selected from formic, acetic, propionic, oxalic, citric, tartaric, malic, fumaric, malonic and maleic acids.

4. A process as claimed in claim 1, wherein the concentration of imiquimod free base in the dispersion ranges from 5 to 15%, and the organic acid carboxylic is added in molar ratio with respect to base ranging from 1:1 to 10:1.

5. A process as claimed in claim 1, wherein the basic agent is an inorganic base.

6. A process as claimed in claim 5, wherein the inorganic base is sodium hydroxide or ammonia.

7. A process as claimed in claim 1, wherein the reaction with the basic agent is carried out in a polar solvent.

8. A process as claimed in claim 1, further comprising a fine grinding or micronisation step, to obtain particles having D[4,3] mean diameter typically equal to or lower than 5 µm.

9. Imiquimod free base with purity of or higher than 99.5%, and with a potentiometric titre ranging from 99 to 101%.

10. Imiquimod free base in the crystalline form having an XRPD spectrum wherein the most intense diffraction peaks fall at 11.04; 17.97; 18.81; 21.24; 21.78; 24.18 in 2θ.

11. Imiquimod free base, as claimed in claim 10, wherein the particles typically have a D[4,3] mean diameter lower than 50 µm.

12. Imiquimod free base, as claimed in claim 10, with purity of or higher than 99.5%, and a potentiometric titre ranging from 99 to 101%.

13. An imiquimod addition salt with an organic mono- or poly-carboxylic acid.

14. An addition salt as claimed in claim 13, wherein the acid is selected from formic, acetic, propionic, oxalic, citric, tartaric, malic, fumaric, malonic and maleic acid.

15. Imiquimod formate in the crystalline form.

16. Imiquimod formate as claimed in claim 15, having an XRPD spectrum wherein the most intense diffraction peaks fall at 5.72; 11.15; 11.54; 17.42; 21.38; 24.95 in 2θ.
